(19) Europäisches Patentamt
European Patent Office
Office européen des brevets

(11) **EP 3 389 600 B1**

(12) **EUROPEAN PATENT SPECIFICATION**

(45) Date of publication and mention
of the grant of the patent:
**02.02.2022  Bulletin 2022/05**

(21) Application number: **16820109.3**

(22) Date of filing: **12.12.2016**

(51) International Patent Classification (IPC):
*A61K 8/23* $^{(2006.01)}$     *A61K 8/02* $^{(2006.01)}$
*A61K 8/81* $^{(2006.01)}$     *A61K 8/19* $^{(2006.01)}$
*A61K 8/365* $^{(2006.01)}$    *A61Q 11/00* $^{(2006.01)}$

(52) Cooperative Patent Classification (CPC):
**A61K 8/022; A61K 8/19; A61K 8/23; A61K 8/365;
A61K 8/8176; A61Q 11/00;** A61K 2800/222;
A61K 2800/31; A61K 2800/882

(86) International application number:
**PCT/US2016/066082**

(87) International publication number:
**WO 2017/106073 (22.06.2017 Gazette 2017/25)**

(54) **FAST DISSOLVING PEROXYMONOSULFATE COMPOSITION**

SCHNELL AUFLÖSENDE PEROXYMONOSULFATZUSAMMENSETZUNG

COMPOSITION DE PEROXYMONOSULFATE À DISSOLUTION RAPIDE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.12.2015  US 201562269488 P**

(43) Date of publication of application:
**24.10.2018  Bulletin 2018/43**

(73) Proprietor: **Colgate-Palmolive Company**
**New York, NY 10022 (US)**

(72) Inventors:
• **LAVENDER, Stacey**
**Chesterfield**
**New Jersey 08515 (US)**
• **XU, Guofeng**
**Plainsboro**
**New Jersey 08536 (US)**
• **DOGO-ISONAGIE, Cajetan**
**Highland Park**
**NJ 08904 (US)**

(74) Representative: **Sonnenhauser, Thomas Martin**
**Wuesthoff & Wuesthoff**
**Patentanwälte PartG mbB**
**Schweigerstraße 2**
**81541 München (DE)**

(56) References cited:
WO-A1-00/09079       WO-A1-99/22705
JP-A- 2007 112 761    US-A- 5 032 178
US-A1- 2014 227 202   US-A1- 2015 306 001
US-B1- 6 274 122      US-B1- 6 596 311

EP 3 389 600 B1

**Description**

**BACKGROUND**

Potassium peroxymonosulfate is a powerful oxidizing and stain removing agent and is currently used in denture cleaning tablets. Typical denture cleaning tablets are quite large weighing about 2.6 g. They are intended to be dissolved in a cup of water (about 200 ml). The dissolve time is on the order of minutes and would be longer in smaller volumes. After dissolving the tablet there still can be some particulates that remain.

Mouthrinses or mouthwashes having tooth whitening effects are desirable. A typical volume of water or aqueous solution for mouthrinse applications is about 15 ml. It would be desirable to have a tablet containing a whitening agent in a solid, e.g., tablet form, which is fast dissolving in water that can be easily used by consumers in mouthrinse applications.

US 2014/227202 A1 discloses an oral care composition comprising at least one carbon dioxide source, at least one acid source, at least one abrasive and one or more pharmaceutically acceptable excipients. The composition can be in the form of fast dissolving powder, or oral rinse.

WO 00/09079 A1 discloses a composition for whitening of teeth, comprising a therapeutically effective amount of a non-peroxide ionic bleaching compound in a mouthrinse, toothpaste, gel dentifrice, chewing gum, wax or lozenge vehicle.

WO 99/22705 A1 discloses denture cleansing compositions, comprising a film-forming oil, an effervescence generator, a foam-forming surfactant, and a water-insoluble, wettable, particulate foam stabiliser.

**BRIEF SUMMARY**

The invention concerns a solid composition, e.g., a tablet or powder, comprising: (a) from 7.5% to 15%, by weight, of an inorganic salt of peroxymonosulfate, (b) a buffering agent, (c) a disintegrating agent comprising crosslinked polyvinylpyrrolidone; and (d) an effervescent couple including 15% to 50% of an effervescent base and 15% to 50% of an effervescent acid, based on the weight of the solid composition; wherein the solid composition completely dissolves in water at 23 °C. The solid composition is quick-dissolving that can be added to water or an aqueous solution to be then used by a consumer as a whitening mouthrinse. Once dissolved the consumer would use the resulting liquid as a typical mouthrinse.

**DETAILED DESCRIPTION**

The following description of the preferred embodiment(s) is merely exemplary in nature and is in no way intended to limit the invention, its application, or uses.

Unless otherwise specified, all percentages and amounts expressed herein and elsewhere in the specification should be understood to refer to percentages by weight. The amounts given are based on the active weight of the material.

Open terms such as "include," "including," "contain," "containing" mean "comprising." In this description, unless otherwise stated, the use of the singular also includes the plural. For example, "a lubricant" also comprehends the case where more than one lubricant is used

The solid composition of the invention comprises peroxymonosulfate.

One embodiment of the peroxymonosulfate is potassium peroxymonosulfate (also known as MPS, potassium monopersulfate). The potassium peroxymonosulfate (an example of which is Oxone®, an oxidizing agent) may be combined to form or exist as a trisalt of potassium peroxymonosulfate, potassium hydrogen sulfate and potassium sulfate $(2KHSO_5 \cdot KHSO_4 \cdot K_2SO_4)$.

Potassium peroxymonosulfate has limited stability in aqueous solutions and in other common toothpaste ingredients. Therefore contact with water during processing and storage should be avoided or minimized. The solid form of the invention overcomes the stability concerns of the oxidizing agent as long as the tablet is packaged in a moisture free environment. The solid composition, e.g., tablet, granules or powder, is individually packaged and sealed in each unit dose.

The solid composition is typically stored in an air tight, moisture-proof package including, e.g., sealed metal foil pouches, blister packs, and desiccant capped tubes. Useful packaging materials include, e.g., polymeric packaging (e.g., polyethylene and polypropylene), metal foils (e.g., aluminum), and combinations thereof.

The composition of the invention completely dissolves in 15 ml of water at 23°C in 1 minute or less with minimal to moderate agitation. The phrase "completely dissolves" means that the resulting solution has no visible particulate material, sediment or solid. The phrase "minimal to moderate agitation" means shaking or swirling of the mixture by hand by the consumer in a suitable container such as a cup or glass. In some embodiments the solid composition dissolves in 50 seconds or less, 40 seconds or less, 30 seconds or less, or 20 seconds or less when added to 15 ml or water or an aqueous solution, e.g., a mouthrinse base.

The solid compositions of the invention contain no water or have a low water content. As used herein, the term "low water content" means the total concentration of water, including any free water and all water contained in any ingredients. In various embodiments of the composition, the amount of water is in an amount of less than 4% by weight, or less than 3% by weight, or less than 2% by weight, or less than 1% by weight, or less than 0.5% by weight, or about 0.001% to about 4% by weight, or about 0.0001% to about 0.5% by weight or about 0.0001% to about 0.1% by weight.

The solid composition of the invention can be in a variety of forms including, e.g., powder (e.g., a free flowing granulation), tablet, caplet (type of tablet), granule, pellet, wafer, film and bead. Typical tablets have an initial hardness (i.e., a hardness immediately after manufacture) of at least 3 kilopounds (Kp), at least 4 Kp, from about 5 Kp to about 15 Kp, or even from about 5 Kp to about 10 Kp, as measured on a standard hardness tester fitted with a strain gauge, and a hardness 24 hours after manufacture of at least 5 Kp, at least 6 Kp, at least 10 Kp, at least 15 Kp, at least 20 Kp, or even from about 30 Kp to about 45 Kp.

The compositions of the invention, e.g., tablets, can be formed to have any desired weight and dimension. Typical composition, e.g., tablet, weights include from 0.05 gram to 5 gram, from 100 milligrams (mg) to 1000 mg, from 100 mg to 500 mg, or even from 200 mg to 400 mg. Useful tablets are also formed with a diameter of at least 5 millimeters (mm), from 5 mm to about 50 mm, from 5 mm to about 30 mm or even from about 5 mm to about 20 mm, and a thickness of at least about 1 mm, from about 1 to about 10mm or about 1 to about 5 mm. In some embodiments the surface area of the compositions, e.g., tablets or beads, can be, for example, about 0.55 to about 9.5 square centimeters ($cm^2$), or about 0.9 $cm^2$ to about 5 $cm^2$.

In certain embodiments, e.g., tablets, the compositions of the invention are layered, e.g., one layer comprising the peroxymonosulfate salt and one or more additional layers comprising other ingredients. In some embodiments the solid compositions, e.g., tablets, are coated with a suitable coating.

The amount of peroxymonosulfate salt, e.g., potassium peroxymonosulfate, in the solid compositions of the invention is effective to result in improved tooth whitening when used twice daily in a mouthrinse for about three months as compared to a control mouthrinse without the peroxymonosulfate salt. The amount of peroxymonosulfate salt is 7.5% to 15%, by weight of the total composition.

The compositions of the invention contain a buffering agent. Examples of buffering agents include anhydrous carbonates such as sodium carbonate, sesquicarbonates, bicarbonates such as sodium bicarbonate, silicates, bisulfates, phosphates such as monopotassium phosphate and dipotassium phosphate, citrates, pyrophosphates (sodium and potassium salts) and combinations thereof. The amount of buffering agent is sufficient to be effective to achieve a pH of about 5 to about 9, preferable about 6 to about 8, and more preferable about 7, when the composition is dissolved in water or a mouthrinse base. In some embodiments the amount of buffering agent is effective to achieve a pH of about 5 to about 10. Typical amounts of buffering agent are about 5 % to about 35%, in one embodiment about 10 % to about 30%, in another embodiment about 15% to about 25%, by weight of the total composition.

The solid compositions of the invention contain a disintegrating agent comprising crosslinked polyvinylpyrrolidone. Additional disintegrating agents include natural starches, such as maize starch, potato starch etc., directly compressible starches such as starch 1500, modified starches such as carboxymethyl starches and sodium starch glycolate which are available as PRIMOJEL® and EXPLOTAB® and EXPLOSOL® and starch derivatives such as amylose. Examples of cross-linked polyvinylpyrrolidones are crospovidones available as e.g. POLYPLASDONE XL® and KOLLIDON XL®. Other disintegrating agents are modified celluloses such as cross-linked sodium carboxymethylcelluloses available as, e.g., AC-DI-SOL®, PRIMELLOSE®, PHARMACEL XL®, EXPLOCEL®, and NYMCEL ZSX®; alginic acid and sodium alginate; microcrystalline cellulose, e.g. AVICEL®, PHARMACEL®, EMCOCELL®, VIVAPUR®; and methacrylic acid-divinylbenzene copolymer salts available as e.g., AMBERLITE® IRP-88. Other examples of the disintegrating agent are light silicic anhydride, calcium silicate, magnesium metasilicate aluminate, and carboxymethyl cellulose. In the present invention, each of them may be used solely or two or more thereof may be used jointly. Typical amounts of disintegrating agent are about 0.5% to about 20%, in one embodiment about 1 % to about 5%, in another embodiment about 1% to about 3%, by weight of the total composition.

The compositions of the invention optionally contain a binder, e.g., when the composition is a tablet, preferably a polymeric binder, that is compatible with an oxidizing agent, which adds bulk to the compositions and assists in holding the components of the composition together when in the form of a tablet. Examples of suitable polymeric binders include, e.g., starches, natural gums, (e.g., xanthan gum), cellulose gums, microcrystalline cellulose, maltodextrins, methylcellulose, cellulose ethers, sodium carboxymethylcellulose, ethylcellulose, gelatin, polyethylene glycol, polyvinylpyrrolidone, pectins, alginates, polyacrylamides, polyvinyloxoazolidone, polyvinyl alcohols and mixtures thereof.

The binder can also comprise one or more non-polymeric binders such as dextrose, lactose, sucrose, sorbitol, mannitol, xylitol and the like.

Typically, the binder is present in the composition in an amount of from 10% by weight to about 60% by weight, from about 15% by weight to about 50% by weight, or even from about 25% by weight to about 40% by weight.

The solid composition of the invention is optionally an effervescent composition. The term "effervescent composition" as used herein means a composition that evolves gas bubbles when contacted with water. When the solid composition of the invention is an effervescent composition, it comprises an effervescent agent. The effervescent agent preferably is an effervescent couple that includes an acid and a base. The effervescent couple is activated when contacted with water, e.g., when the composition, e.g., tablet, is placed in a glass of water. The water liberates the acid and base and enables the acid and base to react with each other to produce carbon dioxide gas, which imparts carbonation to the aqueous composition. Examples of useful acids include citric acid, ascorbic acid, malic acid, adipic acid, tartaric acid, fumaric, succinic acid, sodium acid pyrophosphate, lactic acid, hexamic acid, and acid salts and acid anhydrides thereof, and mixtures thereof. Examples of useful acid anhydrides include citraconic anhydride, glucono-D-lactone, and succinic anhydride. Examples of useful acid salts include potassium bitartrate, acid citrate salts, sodium dihydrogen phosphate, disodium dihydrogen phosphate, sodium acid sulfite, and combinations thereof. The composition includes an effervescent couple including 15% to 50% of an effervescent base and 15% to 50% of an effervescent acid, based on the weight of the solid composition, or even from 25% by weight to 40% by weight of an effervescent acid.

The base preferably is capable of generating carbon dioxide. Examples of suitable carbonate bases include sodium bicarbonate, sodium carbonate, sodium sesquicarbonate, potassium carbonate, potassium bicarbonate, calcium carbonate, magnesium carbonate, magnesium oxide, sodium glycine carbonate, L-lysine carbonate, arginine carbonate, zinc carbonate, zinc oxide and mixtures thereof. The base can be present in the composition even in an amount of from 25% by weight to about 40% by weight.

The compositions of the invention optionally contain a lubricant, e.g., when the composition is a tablet or powder. Various lubricants are suitable for use in the composition including water dispersible, water soluble, water insoluble lubricants and combinations thereof. Examples of useful water soluble lubricants include sodium benzoate, polyethylene glycol, L-leucine, adipic acid, and combinations thereof. The composition can also include water insoluble lubricants including, e.g., stearates (e.g., magnesium stearate, calcium stearate and zinc stearate), oils (e.g., mineral oil, hydrogenated and partially hydrogenated vegetable oils, and cotton seed oil) and combinations thereof. Other water insoluble lubricants include, e.g., animal fats, polyoxyethylene monostearate, talc, and combinations thereof. When the composition is in the form of a tablet, the composition preferably includes a sufficient amount of lubricant to enable the composition to be formed into tablets and released from a high speed tableting press in the form of a tablet. Typically the amount of lubricant in the composition is from 1% by weight to about 15% by weight, from about 1% by weight to about 12% by weight, from about 2% by weight to about 10% by weight, or even from about 3% by weight to about 8% by weight. In one embodiment the composition includes sodium benzoate in an amount of from 1% by weight to about 3% by weight and polyethylene glycol in an amount of from 1% by weight to about 5.5% by weight.

The solid composition of the invention can optionally contain whitening agents in addition to the peroxymonosulfate salt. Whitening agents, material which is effective to effect whitening of a tooth surface to which it is applied, such as hydrogen peroxide and urea peroxide. In various embodiments, the compositions of this invention may optionally comprise a peroxide whitening agent, comprising a peroxide compound. A peroxide compound is an oxidizing compound comprising a bivalent oxygen-oxygen group. Peroxide compounds include peroxides and hydroperoxides, such as hydrogen peroxide, peroxides of alkali and alkaline earth metals, organic peroxy compounds, peroxy acids, pharmaceutically-acceptable salts thereof, and mixtures thereof. Peroxides of alkali and alkaline earth metals include lithium peroxide, potassium peroxide, sodium peroxide, magnesium peroxide, calcium peroxide, barium peroxide, and mixtures thereof. Organic peroxy compounds include carbamide peroxide (also known as urea hydrogen peroxide), glyceryl hydrogen peroxide, alkyl hydrogen peroxides, dialkyl peroxides, alkyl peroxy acids, peroxy esters, diacyl peroxides, benzoyl peroxide, and monoperoxyphthalate, and mixtures thereof. Peroxy acids and their salts include organic peroxy acids such as alkyl peroxy acids, and monoperoxyphthalate and mixtures thereof, as well as inorganic peroxy acid salts such as persulfate, dipersulfate, percarbonate, perphosphate, perborate and persilicate salts of alkali and alkaline earth metals such as lithium, potassium, sodium, magnesium, calcium and barium, and mixtures thereof. In various embodiments, the peroxide compound comprises hydrogen peroxide, urea peroxide, sodium percarbonate and mixtures thereof. In some embodiments, the peroxide compound comprises hydrogen peroxide. In some embodiments, the peroxide compound consists essentially of hydrogen peroxide. In some embodiments a non-peroxide whitening agent may be provided. Whitening agents among those useful herein include non-peroxy compounds, such as chlorine dioxide, chlorites and hypochlorites. Chlorites and hypochlorites include those of alkali and alkaline earth metals such as lithium, potassium, sodium, magnesium, calcium and barium. Non-peroxide whitening agents also include colorants, such as titanium dioxide and hydroxyapatite. One or more additional whitening agents are optionally present in a tooth-whitening effective total amount. In some embodiments the compositions additionally comprise an activator, e.g., tetraacetylethylenediamine.

The solid composition optionally can also include other ingredients including, e.g., flavor agents; fillers; surfactants; preservatives, e.g., sodium benzoate and potassium sorbate; color agents including, e.g., dyes and pigments; and sweeteners.

Examples of the surfactant that can be used are sodium lauryl sulfate, sorbitan fatty acid ester, polyoxyethylene (20) sorbitan monooleate (Polysorbate 80 or Tween 80), polyethylene glycol fatty acid ester, polyoxyethylene sorbitan fatty acid ester, polyoxyethylene alkyl ether, polyoxyethylene polyoxypropylene alkyl ether, polyoxyethylene polyoxypropylene block copolymer, polyoxyethylene alkyl phenyl ether, polyoxyethylene castor oil, polyoxyethylene hydrogenated castor oil, polyoxyethylene sorbitol fatty acid ester and polyoxyethylene glycerol fatty acid ester. In the present invention, each of them may be used solely or two or more thereof may be used jointly. Typical amounts of surfactant are about 0.5 % to about 3%, in one embodiment about 0.75 % to about 2%, in another embodiment about 1% to about 1.5%, by weight of the total composition.

Examples of the filler are crystalline cellulose, ethylcellulose, dextrin, various kinds of cyclodextrin (a-cyclodextrin, β-cyclodextrin and γ-cyclodextrin), sodium sulfate, as well as derivatives thereof and pullulan.

Useful flavor agents include natural and synthetic flavoring sources including, e.g., volatile oils, synthetic flavor oils, flavoring aromatics, oils, liquids, oleoresins and extracts derived from plants, leaves, flowers, fruits, stems and combinations thereof. Useful flavor agents include, e.g., citric oils, e.g., lemon, orange, grape, lime and grapefruit, fruit essences including, e.g., apple, pear, peach, grape, strawberry, raspberry, cherry, plum, pineapple, apricot, and other fruit flavors. Other useful flavor agents include, e.g., aldehydes and esters (e.g., benzaldehyde (cherry, almond)), citral, i.e., alpha-citral (lemon, lime), neral, i.e., beta-citral (lemon, lime), decanal (orange, lemon), aldehyde C-8 (citrus fruits), aldehyde C-9 (citrus fruits), aldehyde C-12 (citrus fruits), tolyl aldehyde (cherry, almond), 2,6-dimethyloctanal (green fruit), 2-dodedenal (citrus, mandarin) and mixtures thereof.

Useful coloring agents include, e.g., food, drug and cosmetic (FD&C) colors including, e.g., dyes, lakes, and certain natural and derived colorants. Useful lakes include dyes absorbed on aluminum hydroxide and other suitable carriers.

Useful sweetening agents include stevia, sugars such as sucrose, glucose, invert sugar, fructose, ribose, tagalose, sucralose, maltitol, erythritol, xylitol, and mixtures thereof, saccharin and its various salts (e.g., sodium and calcium salt of saccharin), cyclamic acid and its various salts, dipeptide sweeteners (e.g., aspartame), acesulfame potassium, dihydrochalcone, glycyrrhizin, and sugar alcohols including, e.g., sorbitol, sorbitol syrup, mannitol and xylitol, and combinations thereof.

It is understood that while general attributes of each of the above categories of materials may differ, there may be some common attributes and any given material may serve multiple purposes within two or more of such categories of materials. All of the ingredients in the compositions may have functions in addition to their primary function, and may contribute to the overall properties of the composition, including its stability, efficacy, consistency, mouthfeel, taste, odor and so forth. For example, a binder may also function as a disintegrating agent and vice versa.

The solid compositions of the invention can be made via techniques known in the art. Documents which disclose techniques which may be used to prepare the solid compositions of the invention are US patents 4,886,669; 6,106,861; 6,596,311; 6,743,443; 6,811,793; 7,501,409; 7,815,897; 8,377,995; and US patent application 2005/0169986. In general, the ingredients and optional components can be kneaded with an organic solvent, filled in a mold and subjected to a compression-molding. The organic solvent can be an alcohol such as methanol, ethanol, propanol, isopropanol, and the like. The kneading and granulating operations carried out by adding such auxiliary agents for making the preparation and by adding such a solvent may be conducted using the conventionally used apparatus. For example, a fluidized bed granulator, a tumbling granulator, an extrusion granulator or a spray-drying drier may be used. The solid compositions may also be prepared via freeze drying.

Powders can be prepared by compounding the ingredients and optionally calcium carbonate, and, if necessary, further orally acceptable additive(s), and mixing in a conventional manner.

Granules can be prepared by any one of known methods for preparing granules such as dry granulation, layering granulation, impregnated-granulation, etc.

For dry granulation, a mixture of ingredients with optional additive(s) is subjected to granulation with a roller compactor, a roll granulator, etc.

For layering granulation, a mixture similar to the above is added to a rolling inactive carriers while spraying a binder solution with a centrifugal fluidized bed granulator or the like to make the mixture adhere to the carries. Examples of the inactive carrier that used in this method include crystals of sugars or inorganic salts such as crystalline lactose, crystalline cellulose, crystalline sodium chloride, etc., and spherical granules such as spherical granules of crystalline cellulose (brand name: Avicel SP, Asahi Kasei Corporation), spherical granules of crystalline cellulose and lactose (brand name: Nonpareil-NP-5 and NP-7, Freund Co., Ltd.), spherical granules of purified white sugar (brand name: Nonpareil-103, Freund Co., Ltd.), spherical granules of lactose and α starch, etc.

For impregnating granulation a solution containing potassium peroxymonosulfate and other ingredients at an appropriate concentration is mixed with porous carriers thereby a sufficient amount of solution is made to retain in the cavities of the carrier, which is followed by drying to remove the solvent. Examples of the porous carrier that can be used include magnesium aluminometasilicate (bland name: Neusiline, Fuji Chemical Industry Co., Ltd.), calcium silicate (Florite, Eisai Co., Ltd.), etc. Examples of the solvent include ethanol, methanol, or the like.

Tablets can be manufactured by either subjecting a mixture prepared in the same manner as above to the compression molding as it is, or subjecting said mixture to the granulation as mentioned above, and then to the compression molding after adding disintegrant(s), lubricant(s), etc., if needed. If a carbonate is compounded, it is preferably added at the same time when a disintegrant, a lubricant, etc. are added. If desired, an additional substance(s) can be compounded.

The compression molding can be conducted using a conventional tableting machine such as rotary tableting machine, single punch tableting machine, dual tableting machine, and the like, with a compressing pressure of generally about 50 to 4,000 kg/cm2.

The present invention also concerns a method for whitening teeth comprising mixing the solid composition of the invention into water or a mouthrinse base until the composition dissolves, followed by rinsing an oral cavity containing teeth with the mouthrinse.

The term "mouthwash" or "mouthrinse" generally denotes liquid formulations which are used to rinse the surfaces of the oral cavity and provide the user with a sensation of oral cleanliness and refreshment. The mouthrinse is an oral composition that is not intentionally swallowed for purposes of systemic administration of therapeutic agents, but is applied to the oral cavity, used to treat the oral cavity and then expectorated. A mouthrinse composition will usually contain an aqueous continuous phase.

A typical mouthrinse composition consists of a liquid carrier such as water, a humectant, such as glycerin, sorbitol, propylene glycol a surfactant, such as a Pluronics, sodium lauryl sulfate, a sweetening agent, such as sodium saccharin, xylitol a flavoring agent, a coloring agent, and a preservative agent, such as potassium sorbate, sodium benzoate. The composition may also include buffering agents that have the capability to buffer to a final pH of 6.5-8, such as sodium phosphates, an anti-cavity agent, such as sodium fluoride, and an antibacterial agent such as cetylpyridinium chloride.

The following non-limiting examples are to illustrate the invention but should not be interpreted as a limitation thereon.

## EXAMPLES

*Example 1*

Effervescent tablet

Table 1: Formula A

| Ingredient | Weight % |
| --- | --- |
| Potassium peroxymonosulfate | 15 |
| Sodium Bicarbonate | 25 |
| Sodium Carbonate | 10 |
| Citric Acid | 25 |
| Crosslinked Polyvinylpyrrolidone | 3 |
| Flavor | 1.9 |
| Polyethylene Glycol | 3 |
| Sodium Lauryl Sulfate | 3 |
| Color | 0.1 |
| Sodium benzoate | 2 |
| Sucralose | 2 |
| Sorbitol | 10 |

*Example 2*

Non-effervescent tablet

Table 2: Formula B

| Ingredient | Weight % |
|---|---|
| Potassium peroxymonosulfate | 15 |
| Sodium Bicarbonate | 25 |
| Sodium Carbonate | 10 |
| Sodium sulfate | 8 |
| Crosslinked Polyvinylpyrrolidone | 3 |
| Flavor | 1.9 |
| Polyethylene Glycol | 3 |
| Sodium Lauryl Sulfate | 3 |
| Color | 0.1 |
| Sodium benzoate | 2 |
| Sucralose | 2 |
| Sorbitol | 10 |

Tablets are prepared using the ingredients of Formula A or B by compression molding.

Example 3

Tablets having the composition of Formula A and Formula B, respectively, are added to a mouthrinse base at 23°C. The mouthrinse base has the following formula:

Table 3

| Ingredient | Weight % |
|---|---|
| Surfactant | 0.1 - 2% |
| Sweetener | 0.0005 - 0.02% |
| Fluoride | 0.01 - 0.05% |
| Preservative | 0.05 - 2% |
| Humectant | 5 - 20% |
| Flavor | 0.05 - 0.6 |
| Color | 0.001 - 0.05% |
| Water | Balance |

Example 4

Whitening efficacy was tested in a mouthwash type setup. MPS was added to an aqueous solution (50 mM phosphate buffer, pH 6.8) and tested versus placebo (buffer alone) and commercial 2% hydrogen peroxide mouthrinse (pH 5.2). The results are shown in Table 4.

Table 4

| | Placebo | 1.0% MPS | 2% HP Rinse |
|---|---|---|---|
| $\Delta W$ | -0.308 $\pm$ 0.50 | -2.849 $\pm$ 0.22 | -3.554 $\pm$ 0.87 |

Results indicate that the MPS results are on par with the 2% hydrogen peroxide rinse even with the short 1 min exposure times. The experiment was conducted at room temperature with shaking. Active oxygen of MPS at each exposure time = 0.047% and theoretical active oxygen of 2% HP = 0.94%.

*Example 5*

Four concentrations of MPS in pH 7.5-8 phosphate buffer are compared to 0.1% hydrogen peroxide in pH 7.5-8 phosphate buffer in an *in vitro* testing procedure using coffee-stained bovine teeth.

The bovine teeth have initial L values of 57-65. The teeth are submerged in deionized water, then removed and soaked for 2 minutes in 20 mL of the test solution. The teeth are then rinsed three times with deionized water. Each such treatment cycle is repeated fourteen times, and after every two cycles values for L*, a* and b* are measured. These figures are used to calculate a whiteness score, W*, which is a measure of overall color change relative to pure white:

$$W* = ((a*)^2 + (b*)^2 + (L*-100)^2)^{\frac{1}{2}}$$

$$\Delta W* = W*(\text{treated}) - W*(\text{baseline})$$

The results are shown in the Table 5 below.

Table 5

| Treatment | 0 | 2 | 4 | 6 | 8 | 10 | 12 | 14 |
|---|---|---|---|---|---|---|---|---|
| 0.5% MPS | 0.00 | 4.13 | 6.06 | 7.90 | 8.49 | 8.74 | 9.08 | 9.04 |
| 1% MPS | 0.00 | 3.76 | 4.41 | 5.78 | 6.53 | 7.04 | 8.15 | 8.27 |
| 2% MPS | 0.00 | 4.59 | 6.26 | 8.07 | 8.67 | 8.85 | 9.14 | 9.26 |
| 0.1% HP | 0 | 0.15 | 0.22 | 0.38 | 0.6 | 0.69 | 0.73 | 1.01 |

0.5% MPS contains substantially the same active oxygen content as 0.1% hydrogen peroxide. Yet, the results demonstrate that all three concentrations of MPS significantly outperform the whitening effect achieved using hydrogen peroxide. Moreover, higher concentrations of MPS are permitted in oral care compositions due to the lower risk of soft tissue irritation, whereas higher concentration of hydrogen peroxide can cause significant irritation of the oral cavity.

*Example 6*

A powdered MPS composition is prepared for use as an additive to a non-whitening or therapeutic mouthwash. The MPS composition contains 47 wt% sodium bicarbonate, 30 wt% sodium carbonate and 23 wt% Caroat whitening agent (containing 47 wt% MPS). This powdered composition can be supplied in a sachet or compressed into a tablet for addition to a mouthwash.

1 gram of the MPS powder formulation is dissolved in 10 mL of the non-whitening mouthwash formulation shown in Table 6 below, resulting in a 1% solution of MPS. The resulting instant whitening mouthwash is compared to a comparative 2% hydrogen peroxide whitening mouthwash composition using the procedure described in Example 5. The comparative composition is shown in Table 7 below. In addition, 1 gram of the MPS powder formulation is also added to 10 mL of the 2% hydrogen peroxide mouthwash for demonstration of an additive effect.

Table 6

| Ingredient | Weight % |
|---|---|
| Water | q.s. |
| Polymers | 0.4 |
| CETYLPYRIDINIUM CHLORIDE | 0.075 |
| Preservative | 0.05 |
| Humectants | 20 |
| Flavor and Color | 0.186 |

(continued)

| Ingredient | Weight % |
|---|---|
| Buffers | 0.022 |

Table 7

| Ingredient | Weight % |
|---|---|
| Water | q.s. |
| Buffers | 0.45 |
| Humectants | 20 |
| Surfactants | 1.0 |
| Hydrogen Peroxide (35%) | 5.71 |
| Polymers | 2.97 |
| Flavor/Sweetener | 0.25 |

The comparative HP mouthwash is compared to the MPS mouthwash and the MPS/HP mouthwash in side-by-side experiments. The results are shown in Tables 8 and 9 below.

Table 8

| Treatment | 0 | 2 | 4 | 6 | 8 | 10 | 12 | 14 |
|---|---|---|---|---|---|---|---|---|
| 1% MPS MW | 0.00 | 2.2 | 3.1 | 4.0 | 4.7 | 5.5 | 6.2 | 6.8 |
| 2% HP MW (sample 1) | 0.00 | 1.1 | 1.5 | 1.9 | 1.9 | 2.1 | 2.3 | 2.3 |

Table 9

| Treatment | 0 | 2 | 4 | 6 | 8 | 10 | 12 | 14 |
|---|---|---|---|---|---|---|---|---|
| 1% MPS/2% HP MW | 0.00 | 3.8 | 5.8 | 6.7 | 7.4 | 8.1 | 8.7 | 8.8 |
| 2% HP MW (sample 2) | 0.00 | 1.1 | 1.7 | 2.4 | 2.5 | 2.9 | 3.2 | 3.5 |

The results demonstrate that an instant 1% MPS mouthwash significantly out-performs the whitening effect achieved using a comparable 2% hydrogen peroxide mouthwash. The results further demonstrate a significant and unexpected additive whitening effect when MPS is added to a hydrogen peroxide mouthwash.

**Claims**

1. A solid composition comprising:

   (a) from 7.5% to 15%, by weight, of an inorganic salt of peroxymonosulfate,
   (b) a buffering agent,
   (c) a disintegrating agent comprising crosslinked polyvinylpyrrolidone; and
   (d) an effervescent couple including 15% to 50% of an effervescent base and 15% to 50% of an effervescent acid, based on the weight of the solid composition;

   wherein the solid composition completely dissolves in water at 23 °C.

2. The composition of claim 1 wherein the composition dissolves in 1 minute or less, 50 seconds or less, 40 seconds or less, 30 seconds or less, or 20 seconds or less, when added to 15 ml of water, at 23 °C.

3. The composition of claim 1 or 2 in the form of a tablet.

4. The composition of any preceding claim wherein the composition contains no water or water in an amount of less than 4% by weight, or less than 3% by weight, or less than 2% by weight, or less than 1% by weight, or less than 0.5% by weight, or about 0.001% to about 4% by weight, or about 0.0001% to about 0.5% by weight or about 0.0001% to about 0.1% by weight.

5. The composition of any preceding claim additionally comprising a binder.

6. The composition of any preceding claim in the form of a tablet having weights from about 0.05 gram to about 5 gram, or from about 100 milligrams (mg) to about 1000 mg, or from about 100 mg to about 500 mg, or from about 200 mg to about 400 mg, a diameter of at least 5 millimeters (mm), or from about 5 mm to about 50 mm, or from about 5 mm to about 30 mm or from about 5 mm to about 20 mm, and a thickness of at least about 1 mm, or from about 1 to about 10mm or from about 1 to about 5 mm.

7. The composition of any preceding claim comprising a polymeric binder wherein the polymeric binder is selected from starches, natural gums, (e.g., xantham gum), cellulose gums, microcrystalline cellulose, maltodextrins, methylcellulose, cellulose ethers, sodium carboxymethylcellulose, ethylcellulose, gelatin, polyethylene glycol, polyvinylpyrrolidone, pectins, alginates, polyacrylamides, polyvinyloxoazolidone, polyvinylalcohols and mixtures thereof; and is present in the composition in an amount of from 10% by weight to about 60% by weight, or from about 15% by weight to about 50% by weight, or from about 25% by weight to about 40% by weight.

8. The composition of any preceding claim wherein the buffering agent is an anhydrous carbonate such as sodium carbonate, a sesquicarbonate, a bicarbonate such as sodium bicarbonate, a silicate, a bisulfate, a citrate, a phosphate such as monopotassium phosphate and dipotassium phosphate, or a combination thereof in an amount of about 5% to about 35%, or about 10 % to about 30%, or about 15% to about 25%, by weight of the total composition.

9. The composition of claim 1 wherein the the effervescent acid is citric acid, ascorbic acid, malic acid, adipic acid, tartaric acid, fumaric, succinic acid, sodium acid pyrophosophate, lactic acid, hexamic acid, citraconic anhydride, glucono-D-lactone, succinic anhydride, potassium bitartrate, acid citrate salts, sodium dihydrogen phosphate, disodium dihydrogen phosphate, sodium acid sulfite, and combinations thereof, and is preferably present in the composition in an amount of from 25% by weight to 40% by weight; and the effervescent base is sodium bicarbonate, sodium carbonate, sodium sesquicarbonate, potassium carbonate, potassium bicarbonate, calcium carbonate, magnesium carbonate, magnesium oxide, sodium glycine carbonate, L-lysine carbonate, arginine carbonate, zinc carbonate, zinc oxide and mixtures thereof, and is preferably present in the composition in an amount of from 25% by weight to 40% by weight.

10. The composition of any preceding claim further comprising a lubricant in an amount from about 1% by weight to about 15% by weight, or from about 1% by weight to about 12% by weight, or from about 2% by weight to about 10% by weight, or from about 3% by weight to about 8% by weight.

11. The composition of any preceding claim additionally comprising one or more additional whitening agents, one or more flavor agents, one or more fillers, one or more surfactants, one or more color agents, or any combination of two or more thereof.

12. A method for whitening teeth comprising mixing the solid composition of claims 1-11 into water or a mouthrinse base until the composition dissolves, followed by rinsing an oral cavity containing teeth with the mouthrinse.

**Patentansprüche**

1. Feste Zusammensetzung, umfassend:

   (a) von 7,5% bis 15%, bezogen auf das Gewicht, eines anorganischen Salzes von Peroxymonosulfat,
   (b) ein Puffermittel,
   (c) ein Desintegrationsmittel, umfassend vernetztes Polyvinylpyrrolidon; und
   (d) ein aufschäumendes Paar, enthaltend 15% bis 50% einer aufschäumenden Base und 15% bis 50% einer aufschäumenden Säure, bezogen auf das Gewicht der festen Zusammensetzung;

wobei sich die feste Zusammensetzung in Wasser bei 23°C vollständig auflöst.

2. Zusammensetzung nach Anspruch 1, wobei sich die Zusammensetzung in 1 Minute oder weniger, 50 Sekunden oder weniger, 40 Sekunden oder weniger, 30 Sekunden oder weniger oder 20 Sekunden oder weniger auflöst, wenn sie bei 23°C zu 15 ml Wasser gegeben wird.

3. Zusammensetzung nach Anspruch 1 oder 2 in Form einer Tablette.

4. Zusammensetzung nach einem beliebigen vorhergehenden Anspruch, wobei die Zusammensetzung kein Wasser oder Wasser in einer Menge von weniger als 4% bezogen auf das Gewicht, oder weniger als 3% bezogen auf das Gewicht, oder weniger als 2% bezogen auf das Gewicht, oder weniger als 1% bezogen auf das Gewicht, oder weniger als 0,5% bezogen auf das Gewicht, oder etwa 0,001% bis etwa 4% bezogen auf das Gewicht, oder etwa 0,0001% bis etwa 0,5% bezogen auf das Gewicht oder etwa 0,0001% bis etwa 0,1% bezogen auf das Gewicht enthält.

5. Zusammensetzung nach einem beliebigen vorhergehenden Anspruch, zusätzlich umfassend ein Bindemittel.

6. Zusammensetzung nach einem beliebigen vorhergehenden Anspruch in Form einer Tablette mit einem Gewicht von etwa 0.05 Gramm bis etwa 5 Gramm, oder von etwa 100 Milligramm (mg) bis etwa 1000 mg, oder von etwa 100 mg bis etwa 500 mg, oder von etwa 200 mg bis etwa 400 mg, einen Durchmesser von mindestens 5 Millimetern (mm), oder von etwa 5 mm bis etwa 50 mm, oder von etwa 5 mm bis etwa 30 mm, oder von etwa 5 mm bis etwa 20 mm, und eine Dicke von mindestens etwa 1 mm, oder von etwa 1 bis etwa 10 mm, oder von etwa 1 bis etwa 5 mm.

7. Zusammensetzung nach einem beliebigen vorhergehenden Anspruch, umfassend ein polymeres Bindemittel, wobei das polymere Bindemittel ausgewählt ist aus Stärken, natürlichen Gummis, (z. B., Xanthamgummi), Cellulosegummis, mikrokristalliner Cellulose, Maltodextrinen, Methylcellulose, Celluloseethern, Natriumcarboxymethylcellulose, Ethylcellulose, Gelatine, Polyethylenglykol, Polyvinylpyrrolidon, Pektinen, Alginaten, Polyacrylamiden, Polyvinyloxoazolidon, Polyvinylalkoholen und deren Mischungen ausgewählt ist; und in der Zusammensetzung in einer Menge von 10% bezogen auf das Gewicht bis zu etwa 60% bezogen auf das Gewicht, oder von etwa 15% bezogen auf das Gewicht bis zu etwa 50% bezogen auf das Gewicht, oder von etwa 25% bezogen auf das Gewicht bis zu etwa 40% bezogen auf das Gewicht vorhanden ist.

8. Zusammensetzung nach einem beliebigen vorhergehenden Anspruch, wobei das Puffermittel ein wasserfreies Carbonat wie Natriumcarbonat, ein Sesquicarbonat, ein Bicarbonat wie Natriumbicarbonat, ein Silicat, ein Bisulfat, ein Citrat, ein Phosphat wie Monokaliumphosphat und Dikaliumphosphat oder eine Kombination davon in einer Menge von etwa 5% bis etwa 35%, oder etwa 10% bis etwa 30%, oder etwa 15% bis etwa 25%, bezogen auf das Gewicht der Gesamtzusammensetzung, ist.

9. Zusammensetzung nach Anspruch 1, wobei die aufschäumende Säure Zitronensäure, Ascorbinsäure, Äpfelsäure, Adipinsäure, Weinsäure, Fumarsäure, Bernsteinsäure, Natriumsäure-Pyrophosophat, Milchsäure, Hexamsäure, Citraconsäureanhydrid, Glucono-D-Lacton, Bernsteinsäureanhydrid, Kaliumbitartrat, saure Citratsalze, Natriumdihydrogenphosphat, Dinatriumdihydrogenphosphat, Natriumsäure-Sulfit und Kombinationen davon, und ist vorzugsweise in der Zusammensetzung in einer Menge von 25% bezogen auf das Gewicht bis 40% bezogen auf das Gewicht vorhanden; und die aufschäumende Base Natriumbicarbonat, Natriumcarbonat, Natriumsesquicarbonat, Kaliumcarbonat, Kaliumbicarbonat, Calciumcarbonat, Magnesiumcarbonat, Magnesiumoxid, Natriumglycincarbonat, L-Lysincarbonat, Arginincarbonat, Zinkcarbonat, Zinkoxid und Mischungen davon ist und vorzugsweise in der Zusammensetzung in einer Menge von 25% bezogen auf das Gewicht bis 40% bezogen auf das Gewicht vorhanden ist.

10. Zusammensetzung nach einem beliebigen vorhergehenden Anspruch, weiterhin umfassend ein Schmiermittel in einer Menge von etwa 1% bezogen auf das Gewicht bis etwa 15% bezogen auf das Gewicht, oder von etwa 1% bezogen auf das Gewicht bis etwa 12% bezogen auf das Gewicht, oder von etwa 2% bezogen auf das Gewicht bis etwa 10% bezogen auf das Gewicht, oder von etwa 3% bezogen auf das Gewicht bis etwa 8% bezogen auf das Gewicht umfasst.

11. Zusammensetzung nach einem beliebigen vorhergehenden Anspruch, zusätzlich umfassend einen oder mehrere zusätzliche aufhellende Mittel, einen oder mehrere Geschmacksstoffe, einen oder mehrere Füllstoffe, ein oder mehrere oberflächenaktive Mittel, einen oder mehrere Farbstoffe oder eine beliebige Kombination von zwei oder mehreren davon umfasst.

**12.** Verfahren zum Aufhellen von Zähnen, umfassend das Mischen der festen Zusammensetzung nach den Ansprüchen 1-11 in Wasser oder einer Mundspülungsbasis, bis sich die Zusammensetzung auflöst, gefolgt von der Spülung einer Mundhöhle, die Zähne enthält, mit der Mundspülung.

**Revendications**

**1.** Composition solide comprenant :

(a) de 7,5 % à 15 %, en poids, d'un sel inorganique de peroxymonosulfate,
(b) un agent tampon,
(c) un agent de désintégration comprenant de la polyvinylpyrrolidone réticulée ; et
(d) un couple effervescent comprenant 15 % à 50 % d'une base effervescente et 15 % à 50 % d'un acide effervescent, par rapport au poids de la composition solide ;

dans laquelle la composition solide se dissout complètement dans l'eau à 23 °C.

**2.** Composition selon la revendication 1, dans laquelle la composition se dissout en 1 minute ou moins, 50 secondes ou moins, 40 secondes ou moins, 30 secondes ou moins, ou 20 secondes ou moins, lorsqu'elle est ajoutée à 15 ml d'eau, à 23 °C.

**3.** Composition selon la revendication 1 ou 2 sous la forme d'un comprimé.

**4.** Composition selon l'une quelconque des revendications précédentes, dans laquelle la composition ne contient pas d'eau ou de l'eau en une quantité inférieure à 4 % en poids, ou inférieure à 3 % en poids, ou inférieure à 2 % en poids, ou inférieure à 1 % en poids, ou inférieure à 0,5 % en poids, ou d'environ 0,001 % à environ 4 % en poids, ou d'environ 0,0001 % à environ 0,5 % en poids ou d'environ 0,0001 % à environ 0,1 % en poids.

**5.** Composition selon l'une quelconque des revendications précédentes comprenant en outre un liant.

**6.** Composition selon l'une quelconque des revendications précédentes sous la forme d'un comprimé ayant des poids d'environ 0,05 gramme à environ 5 grammes, ou d'environ 100 milligrammes (mg) à environ 1 000 mg, ou d'environ 100 mg à environ 500 mg, ou d'environ 200 mg à environ 400 mg, un diamètre d'au moins 5 millimètres (mm), ou d'environ 5 mm à environ 50 mm, ou d'environ 5 mm à environ 30 mm ou d'environ 5 mm à environ 20 mm, et une épaisseur d'au moins environ 1 mm, ou d'environ 1 à environ 10 mm ou d'environ 1 à environ 5 mm.

**7.** Composition selon l'une quelconque des revendications précédentes, comprenant un liant polymérique dans laquelle le liant polymérique est choisi parmi les amidons, les gommes naturelles, (par exemple, la gomme xanthane), les gommes de cellulose, la cellulose microcristalline, les maltodextrines, la méthylcellulose, les éthers de cellulose, la carboxyméthylcellulose sodique, l'éthylcellulose, la gélatine, le polyéthylène glycol, la polyvinylpyrrolidone, les pectines, les alginates, les polyacrylamides, la polyvinyloxoazolidone, les alcools polyvinyliques et leurs mélanges ; et est présent dans la composition en une quantité de 10 % en poids à environ 60 % en poids, ou d'environ 15 % en poids à environ 50 % en poids, ou d'environ 25 % en poids à environ 40 % en poids.

**8.** Composition selon l'une quelconque des revendications précédentes, dans laquelle l'agent tampon est un carbonate anhydre tel que le carbonate de sodium, un sesquicarbonate, un bicarbonate tel que le bicarbonate de sodium, un silicate, un bisulfate, un citrate, un phosphate tel que le phosphate monopotassique et le phosphate dipotassique, ou une combinaison de ceux-ci en une quantité d'environ 5 % à environ 35 %, ou d'environ 10 % à environ 30 %, ou d'environ 15 % à environ 25 %, en poids de la composition totale.

**9.** Composition selon la revendication 1, dans laquelle l'acide effervescent est l'acide citrique, l'acide ascorbique, l'acide malique, l'acide adipique, l'acide tartrique, l'acide fumarique, l'acide succinique, le pyrophosphate acide de sodium, l'acide lactique, l'acide hexamique, l'anhydride citraconique, la glucono-D-lactone, l'anhydride succinique, le bitartrate de potassium, les sels de citrate acide, le dihydrogénophosphate de sodium, le dihydrogénophosphate disodique, le sulfite acide de sodium et leurs combinaisons, et est de préférence présent dans la composition en une quantité de 25 % en poids à 40 % en poids ; et la base effervescente est le bicarbonate de sodium, le carbonate de sodium, le sesquicarbonate de sodium, le carbonate de potassium, le bicarbonate de potassium, le carbonate de calcium, le carbonate de magnésium, l'oxyde de magnésium, le carbonate de glycine de sodium, le carbonate de L-lysine, le carbonate d'arginine, le carbonate de zinc, l'oxyde de zinc et leurs mélanges, et est de préférence présent dans la composition en une quantité de 25 % en poids à 40 % en poids.

**10.** Composition selon l'une quelconque des revendications précédentes, comprenant en outre un lubrifiant en une quantité d'environ 1 % en poids à environ 15 % en poids, ou d'environ 1 % en poids à environ 12 % en poids, ou d'environ 2 % en poids à environ 10 % en poids, ou d'environ 3 % en poids à environ 8 % en poids.

**11.** Composition selon l'une quelconque des revendications précédentes, comprenant en outre un ou plusieurs agents de blanchiment supplémentaires, un ou plusieurs agents aromatisants, une ou plusieurs charges, un ou plusieurs tensioactifs, un ou plusieurs agents colorants, ou toute combinaison de deux ou plus de ceux-ci.

**12.** Procédé de blanchiment des dents comprenant le mélange de la composition solide des revendications 1 à 11 dans de l'eau ou une base de bain de bouche jusqu'à ce que la composition se dissolve, suivi du rinçage d'une cavité buccale contenant des dents avec le bain de bouche.

**EP 3 389 600 B1**

**REFERENCES CITED IN THE DESCRIPTION**

**Patent documents cited in the description**

- US 2014227202 A1
- WO 0009079 A1
- WO 9922705 A1
- US 4886669 A
- US 6106861 A
- US 6596311 B
- US 6743443 B
- US 6811793 B
- US 7501409 B
- US 7815897 B
- US 8377995 B
- US 20050169986 A